# EUROPÄISCHE PATENTSCHRIFT

(11) **EP 0 574 434 B1**
(45) Veröffentlichungstag und Bekanntmachung des Hinweises auf die Patenterteilung: **26.07.1995**
(21) Anmeldenummer: 92905271.0
(22) Anmeldetag: 29.02.1992
(51) Int. Cl.: A61M 37/00

(54) **KANÜLE ZUM ABLEGEN EINES ELEMENTS**
NEEDLE FOR INSERTING AN OBJECT INTO THE BODY
CANULE POUR L'INSERTION D'UN ELEMENT

(30) Priorität: 06.03.1991 DE 4107042; 15.10.1991 DE 9112797 U; 18.12.1991 DE 9115658 U
(43) Veröffentlichungstag der Anmeldung: 22.12.1993
(73) Patentinhaber: SÜDDEUTSCHE FEINMECHANIK GMBH, D-63607 Wächtersbach (DE)
(72) Erfinder: HIRSCH, Christian, D-63486 Bruchköbel (DE); FEIT, Herbert, D-63607 Wächtersbach (DE)
(74) Vertreter: Stoffregen, Hans-Herbert, Dr. Dipl.-Phys.
(86) Internationale Anmeldenummer: EP9200444
(87) Internationale Veröffentlichungsnummer: WO9215362

(56) Entgegenhaltungen:
- EP-A- 0 292 936
- WO-A-90/05488
- DE-A- 1 223 495
- US-A- 4 820 267

## Beschreibung

Die Erfindung bezieht sich auf eine Kanüle mit am vorderen Ende angeschliffener Spitze und am hinteren Ende die Kanüle haltendem Kanülenhalter, einem in der Kanüle verschiebbaren Mandrin sowie gegebenenfalls einer lösbar mit dem Kanülenhalter verbundenen, die Kanülenspitze umgebenden Schutzkappe vorzugsweise in Form eines einseitig offenen Hohlzylinders, wobei die Kanüle bestimmt ist zum Ablegen eines Elementes in einem Körper eines Lebewesens durch Wechselwirken mit und Relativbewegung zwischen dem Mandrin und der Kanüle, wobei das Element vor Ablegen in dem Körper zwischen dem Mandrin und der Kanülenspitze lagefixiert ist.

Es ist bekannt, zur Identifikation von Tieren elektronische Identifikationsträger wie Mikrotransponder oder Chips zu implantieren. Hierzu wird unter dem Hautgewebe der Tiere der elektronische Identifikationsträger abgelegt. Dies erfolgt mit einer Kanüle, in der der Transponder verschiebbar angeordnet ist. Die Kanüle wird zunächst unter das Hautgewebe geschoben. Sodann wird die Kanüle langsam zurückgezogen, wobei ein innerhalb der Kanüle vorhandener und zu dieser verschiebbarer Mandrin bewirkt, daß beim Zurückziehen der Kanüle der Transponder oder der Chip im Tierfleisch abgelegt wird. Die Kanüle wird folglich gegenüber dem quasi stationären Mandrin bewegt. Hierzu kann eine pistolenartige Handhabe benutzt werden, wie sie z. B. der GB-A-2 190 590 zu entnehmen ist. Zum Zeitpunkt des abgelegten Identifikationsträgers befindet sich die vordere Spitze des Mandrins in oder vor der Öffnung der Kanüle, so daß der Mandrin mit dem Gewebe bsw. Blut des Lebewesens in Berührung kommt. Dabei besteht auch die Gefahr, daß sich das Gewebe entzündet.

Kanülen zum Ablegen von festen oder halbfesten Präparaten unter die Haut eines Menschen sind z. B. der US-A-4,900,304, US-A-4,950,234 oder EP-A-0 255 123 zu entnehmen. In der EP-A-0 304 107 ist eine Injektionskanüle zum Ablegen eines Implantats beschrieben. Die Kanüle ist zum einmaligen Gebrauch bestimmt.

Aus der US-A-4,820,267 ist eine Kanüle der eingangs genannten Art zum Ablegen eines Implantats bekannt. Zu Transportzwecken ist das Implantat proximal von einem Mandrin und distal von einem Stift lagefixiert. Um sicherzustellen, daß der Stift nicht unkontrolliert aus der Kanüle herausrutscht, wird dieser außenseitig von einer Schutzhülle festgelegt. Während des Implantierens wird der Stift entfernt, da anderenfalls die Kanüle nicht einsatzbereit wäre.

Zur Identifizierung von Tieren ist nach der WO 90/05488 eine flexible Kanüle vorgesehen, dessen distaler Bereich einen geringeren Querschnitt als derjenige aufweist, in dem ein Mandrin verschiebbar ist. Eine distale Lagefixierung besteht nicht.

Um in tierischen Körpern Hormonpräparate subkutan zu implantieren, wird nach der DE-B-1 223 495 ein eine Hohlnadel tragender rohrförmiger Körper vorgesehen, in dessen Hohlraum eine die Präparate enthaltende Hülse einsetzbar ist. Lagefixierungen innerhalb der Hülse selbst sind nicht vorgesehen.

Bei der aus der EP-A-0 292 936 bekannten Vorrichtung zum Implantieren von Präparaten werden letztere von einem Führungselement aufgenommen, von dem seinerseits eine Kanüle ausgeht. Eine entsprechende Vorrichtung ist im Vergleich zu den Kanülen, in denen selbst das Präparat lagefixiert wird, konstruktiv aufwendig. Außerdem bedarf es eines besonderen Geschicks des Benutzers, da über die gesamte Kanülenlänge das Präparat verschoben werden muß, bevor es abgelegt wird.

Grundsätzlich befindet sich die mit der Handhabe verbindbare Kanüle in einer an und für sich bekannten sterilen Verpackung, die bei der Benutzung der Kanüle zunächst aufgerissen werden muß, um sodann die Kanüle entnehmen zu können. Durch die Art der Verpackung bedingt fällt im Vergleich zur G Größe der Kanüle relativ viel Verpackungsmaterial an. Auch ist das Verpacken und Auspacken aufwendig.

Der vorliegenden Erfindung liegt das Problem zugrunde, eine Kanüle der eingangs beschriebenen Art so weiterzubilden, daß sie problemlos und insbesondere ohne aufwendige oder teure Hilfsmittel benutzt werden kann. Dabei soll gewährleistet sein, daß sich das Element in einer vorher bekannten Position innerhalb der Kanüle befindet, um so das Implantieren zu vereinfachen und das Ablegen in einer gewünschten Gewebetiefe sicherzustellen. Auch soll das Element innerhalb der Kanüle, unabhängig von Schutzkappe oder Verpackungen, eindeutig lagefixierbar sein.

Ferner soll sichergestellt sein, daß nicht die Gefahr besteht, daß Lebewesen bei der Verwendung der Kanüle infiziert werden.

Schließlich sollen ein universeller Einsatz und Vereinfachungen bei der Verpackung der sterilen Kanüle gegeben sein sowie der Anfall an Abfall reduziert werden.

Das Problem wird erfindungsgemäß im wesentlichen dadurch gelöst, daß das Element kanülenspitzenseitig von einem die Kanüle zumindest bereichsweise verschließenden Verschluß in Form eines einen medizinischen Wirkstoff enthaltenden Materials, eines Klebstoffmaterials oder eines Silikonpfropfens und/oder halterungsseitig durch eine Querschnittsänderung der Kanüle lagefixiert ist.

Erfindungsgemäß ist sowohl distal als auch proximal eine eindeutige Lagefixierung des Elementes gewährleistet, ohne daß weder eine Schutzkappe noch der Mandrin selbst benötigt wird. Dabei ist der distale Verschluß kein starres Element. Vielmehr handelt es sich um ein flexibles Material, das einen medizinischen Wirkststoff enthalten kann, der beim Ablegen zumindest bereichsweise an dem Element haften bleibt. Hierdurch ist zumindest eine Vorbeugung gegen Entzündungen gegeben. Der Verschluß übt folglich eine Doppelfunktion aus.

Als Verschluß kann aber auch z. B. ein Klebstoffmaterial oder ein Silikonpfropfen dienen.

Halterungsseitig erfolgt die Lagefixierung durch eine Querschnittsänderung wie -verringerung des Lumens.

Die Querschnittsänderung dient gleichzeitig als Schiebewegbegrenzung für den der. Kanülenspitze abgewandten Endbereich des Mandrins. Hierdurch ist sichergestellt, daß der Mandrin mit seinem hinteren Ende nur bis zu der Querschnittsverringerung verschoben werden kann, so daß beim Entfernen der Kanüle aus dem Gewebe sichergestellt ist, daß der Mandrin stets in der Kanüle verbleibt.

Die Querschnittsänderung selbst kann z. B. durch zumindest zwei diametral zur Längsachse der Kanüle verlaufende Einbuchtungen wie Körnungen gebildet sein, wodurch zusätzlich eine Führung des Mandrins innerhalb der Kanüle erfolgt. Hierdurch ist sichergestellt, daß der Mandrin, der vorzugsweise aus Kunststoff besteht, nicht innerhalb der Kanüle unkontrolliert verbogen wird.

Anstelle von Einbuchtung und Körnung oder ähnliches kann eine Querschnittsverringerung auch durch eine umlaufende, gegebenenfalls nur abschnittsweise umlaufende Sicke gebildet werden.

Durch die Lagefixierung zwischen Verschluß und Querschnittsänderung ist folglich sichergestellt, daß insbesondere beim Transport ein unkontrolliertes oder unerwünschtes langes Hin- und Herrutschen des Elementes innerhalb der Kanüle unterbleibt.

Zu dem kanülenspitzenseitigen Verschluß ist noch auf folgendes zu verweisen. Bei der Verwendung von Klebstoffmaterial sollte dieses in bezug auf die Kanüle eine Adhäsion aufweisen, die bei Durchdringen des Materials von dem Element bzw. dem Mandrin sicherstellt, daß das Klebstoffmaterial in der Kanüle haften bleibt.

Das Verschlußmaterial, welches hervorzuhebenderweise berührungslos in die Kanüle durch z. B. Eintropfen einbringbar ist, sollte aufgrund der Adhäsion zumindest eine ringförmige Querschnittsverringerung des Lumens der Kanüle bewirken, wobei gegebenenfalls im Längsachsenbereich ein hautförmig ausgebildeter Verschluß ausgebildet ist, wodurch das Element nach außen hin vollständig abgeschirmt ist.

Eine weitere Ausgestaltung der Erfindung sieht des weiteren vor, daß zum Verschieben des Mandrins in Richtung der Kanülenspitze in die Kanüle ein Stiftelement wie Stahlstift einführbar ist, der seinerseits vorzugsweise von der die Kanüle umgebenden Schutzkappe ausgeht.

Die Schutzkappe kann nach einem weiteren Vorschlag der Erfindung ein einseitig offener Hohlzylinder sein, wobei der Innendurchmesser der Zylinderkappe dem Außendurchmesser des Kanülenhalters entspricht. Beim Implantieren des Elements wird der Kanülenhalter innerhalb der Schutzkappe verschoben, wodurch der von der Bodenfläche der Schutzkappe abragende Stift in die Kanüle eindringt, um somit beim Zurückziehen der Kanüle aus dem Gewebe, also beim Verschieben der Kanüle in Richtung der Bodenfläche der Schutzkappe den Mandrin in Richtung der Kanülenspitze zu verschieben wild, um So sicherzustellen, daß das Element im Gewebe abgelegt werden kann.

Sofern die Schutzkappe die eigentliche Funktion der Schutzkappe ausüben soll, durchsetzt die Kanüle von der Außenseite der Bodenfläche diese, und zwar in einer Durchbrechung, die außermittig verläuft. In diesen, Fall verläuft die Kanüle parallel zu dem Stiftelement und erstreckt sich innerhalb der Schutzkappe, die zusammen mit der Handhabe z. B. in einem Verpackungsmaterial eingschweißt sein kann.

Um das Zurückziehen der Kanüle kontrolliert durchzuführen, können von dem Kanülenhalter stegartige Handhaben abragen, die in Längsschlitzen der Schutzkappe führbar sind. Um ein unkontrolliertes Herausrutschen der Kanüle aus der Handhabe auszuschließen, ist der jeweilige Längsschlitz vorzugsweise gestuft ausgebildet.

Nach einem selbständigen Lösungsvorschlag ist vorgesehen, daß der Kanülenhalter im sich an die Kanüle anschließenden Bereich eine hohlzylindrische Erweiterung aufweist, an deren Innendurchmesser ein tellerförmig ausgebildeter Abschnitt des Mandrins angepaßt ist, und daß die hohlzylindrische Erweiterung zur verschiebbaren Aufnahme eines Schiebeelements ausgebildet ist, wobei das Schiebeelement vorzugsweise die Schutzkappe selbst ist.

Vorteilhaft ausgestaltend wird eine Kanüle zum Implantieren von Elementen vorgschlagen, bei der der Mandrin als Kolben oder Stößel ausgebildet ist, der in dem die Funktion eines Zylinders aufweisenden Kanülenhalter mittels des gleichfalls in die zylindrische Erweiterung einbringaren Schiebeelements beaufschlagbar ist. Durch diese Maßnahmen kann der Mandrin ohne große Kraftanstrengungen und bei Verwendung der Schutzkappe als Schiebeelement ausschließlich mit den für die Kanüle notwendigen Elementen in ein Gewebe geschoben werden. Dabei wird wie bei den bekannten Kanülen die Kanüle aus dem Gewebe gezogen, um bei quasi stationärem Verharren des Mandrins den Identifikationsträger im Fleisch zu implantieren.

Damit die Schutzkappe problemlos in die hohlzylindrische Erweiterung des Kanülenträgers eingebracht werden kann, ist das geschlossene, also die Kanülenspitze umgebende Ende im Vergleich zu dem übrigen dem Innendurchmesser der Erweiterung angepaßten Abschnitt verjüngt ausgebildet.

Um ein Wechselwirken zwischen der Schutzkappe und dem Kolben, also dem tellerförmig ausgebildeten Abschnitt des Mandrins sicherzustellen, ist das geschlossene Ende der Schutzkappe zumindest bereichsweise komplementär zu der außenliegenden Fläche des tellerförmigen Abschnitts ausgebildet. Vorzugsweise weist dabei die Schutzkappe eine plane Stirnfläche auf, so daß folglich auch die außen liegende Fläche des die Kolbenfunktion ausübenden tellerförmigen Abschnitts des Mandrins plan ist.

Ein problemloses Hineindrücken der Schutzkappe in die hohlzylindrische Erweiterung des Kanülenhalters ist dadurch ermöglicht, daß die die Öffnungen von Schutzkappe bzw. Kanülenhalter umgebenden Ränder nach außen abgewinkelte Abschnitte aufweisen.

Um eine Vereinfachung der Verpackung der Kanüle und eine Verringerung von Abfall zu erreichen, ist vorgesehen, daß sich die Kanüle vollständig innerhalb der Schutzkappe erstreckt, die an ihrem offenen Ende mit einer flexiblen Abdeckung versehen ist, daß die Schutzkappe zumindest zwei Abschnitte unterschiedlicher Querschnitte aufweist, von denen ein vorderer (erster) die Kanülenspitze umgebender Abschnitt mit seinem der Kanülenspitze abgewandten Bereich auf dem Kanülenhalter angeordnet ist, und daß ein endseitiger (zweiter) Abschnitt einerseits mit der flexiblen Abdeckung verschlossen ist und andererseits abtrennbar mit dem ersten Abschnitt verbunden ist.

Als weitere vorteilhafte Ausgestaltung dient folglich die Schutzkappe nicht nur als Schutz für die Kanülenspitze sondern gleichzeitig als sterile Verpackung, so daß sowohl beim Verpacken der Kanüle selbst als auch beim Auspacken dieser Vereinfachungen gegeben sind. Insbesondere erübrigt sich der ansonsten notwendige Verpackungsvorgang, da mit dem Aufbringen der Schutzkappe die Verpackung selbst zur Verfügung gestellt ist. Es ist dann nur noch erforderlich, daß die offene Stirnseite, die im Bereich des Kanülenhalter verläuft, mit einer flexiblen Abdeckung wie Papier verschlossen wird.

Bei der Benutzung der Kanüle ergibt sich der Vorteil, daß im Vergleich zu den ansonsten bekannten Verpackungen nur noch die Abdeckung der Schutzkappe oder der hintere Abschnitt der Schutzkappe als Abfall anfällt, wohingegen der verbleibende Teil der Verpackung, also die Schutzkappe selbst in gewohnter Weise gehandhabt wird.

Insbesondere aus Gründen der Materialersparnis kann der vordere Abschnitt der Schutzkappe seinerseits aus Bereichen unterschiedlicher Querschnitte zusammengesetzt sein, wobei der den Kanülenhalter umgebende Bereich im Vergleich zum die freie Kanüle umgebenden Bereich einen größeren Querschnitt aufweist.

Um die Kanüle in gewohnter Weise zu handhaben, ist des weiteren vorgesehen, daß einerseits der zweite Abschnitt beabstandet den Kanülenhalter umgibt und andererseits zwischen dem ersten und dem zweiten Abschnitt eine Sollbruchstelle ausgebildet ist.

Dabei kann vom dem ersten Abschnitt zugewandten Stirnbereich des zweiten Abschnitts eine den ersten mit dem zweiten Abschnitt verbindende stegartige Wandung ausgehen.

Alternativ besteht die Möglichkeit, daß im Verbindungsbereich zwischen dem ersten und dem zweiten Abschnitt der Außendurchmesser des ersten Abschnitts in etwa dem Innendurchmesser des zweiten Abschnitts entspricht, wodurch sich eine umlaufende Abreißkante ausbildet.

Nach einem weiteren Vorschlag der Erfindung kann im Übergangsbereich zwischen dem ersten und dem zweiten Abschnitt im ersten Abschnitt eine umlaufende Einkerbung zur Ausbildung einer Sollbruchstelle verlaufen.

Die erfindungsgemäße Kanüle ist nicht nur für den veterinärmedizinischen Einsatz bestimmt. Vielmehr ist auch eine Verwendung im humanmedizinischen Bereich möglich. Hierzu kann das Element z. B. als medizinisches Langzeitpräparat, als radioaktive Substanzen enthaltende Kapsel (Slaks) oder als metallisches Kontrastelement ausgebildet sein oder enthalten.

In bezug auf medizinische Langzeitpräparate sind z.B. Antibabypräparate oder Morphiumpräparate denkbar.

Durch die erfindungsgemäße Verwendung ergibt sich insbesondere in bezug auf die radioaktive Substanzen enthaltenden Kapseln der Vorteil, daß ein gezielteres Implantieren in den Körperbereichen erfolgen kann, die behandelt werden sollen.

Weitere Einzelheiten, Vorteile und Merkmale der Erfindung ergeben sich nicht nur aus den Ansprüchen, den diesen zu entnehmenden Markmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: eine Schnittdarstellung durch eine Kanüle zum Implantieren von Elementen.
- Fig. 2: eine Schnittdarstellung eines Implantators mit einer Kanüle gemäß Fig. 1,
- Fig. 3: eine Ansicht entlang der Linie III-III in Fig. 2,
- Fig. 4: einen vergrößerten Ausschnitt der Kanüle nach Fig. 1,
- Fig. 5: eine weitere Ausführungsform eines Implantators,
- Fig. 6: eine weitere Ausführungsform einer Kanüle zum Implantieren eines Elementes in einem Gewebe mit einer die Kanüle umgebenden Schutzkappe, im Schnitt,
- Fig. 7: einen Ausschnitt der Schutzkappe nach Fig. 6 und
- Fig. 8 und 9: Ausschnitte weiterer Ausführungsformen von Schutzkappen.

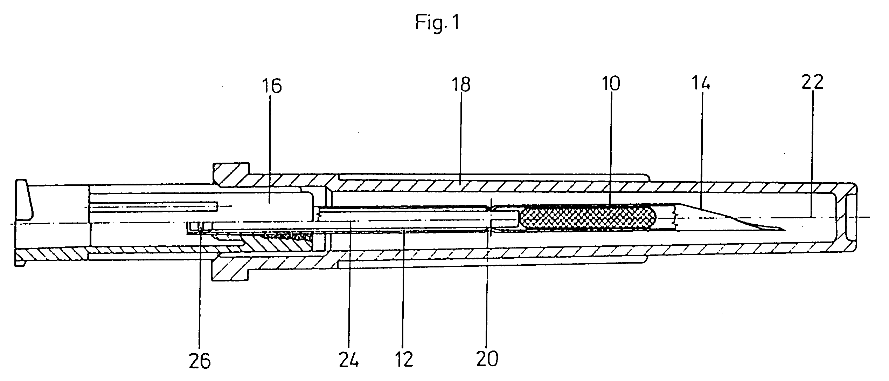

Um in einem tierischen oder menschlichen Lebewesen ein Element wie Identifikationsträger, medizinisches Langzeitpräparat, Kontrastmetall oder ein eingekapseltes radioaktives Präparat (Slak), das in der Fig. 1 ganz allgemein mit dem Bezugszeichen (10) versehen ist, zu implantieren, wird eine Kanüle (12) benutzt.

Bei der Kanüle (12) kann es sich um eine Stahlkanüle, Kunststoffkanüle oder um eine Kanüle anderen geeigneten Materials mit schräg angeschliffener Spitze (14) handeln. Die Kanüle (12) wird von einem Kanülenhalter (16) aufgenommen. Von dem Kanülenhalter (16) geht lösbar eine Schutzkappe (18) aus, die die Kanüle (12) umgibt.

Die Kanüle weist im Abstand zur Spitze (14) und der Kanülenhalterung (16) eine Lumeneinschnürung (20) auf, die z. B. durch in bezug auf die Kanülenlängsachse (22) zwei diametral angeordnete Körnungen oder eine zumindest bereichsweise umlaufende Sicke gebildet werden kann. Der Querschnitt im Bereich der Einschnürung (20) ist dabei geringer als der Durchmesser des Identifikationsträgers (10), wodurch sichergestellt ist, daß dieser über die Einschnürung (20) hinaus nicht in Richtung der Kanülenhalterung (16) verrutschen kann.

In der Kanüle (12) ist des weiteren ein Mandrin (24) verschiebbar angeordnet, und zwar von der Kanülenhalterungsseite (16) her. Der Mandrin hat im der Kanülenspitze (14) abgewandtem Endbereich einen verstärkten Querschnitt wie umlaufenden Wulst (26), dessen Durchmesser größer als der der Einschnürung (20) ist. Hierdurch ist sichergestellt, daß die Verstärkung (26) die Einschnürung (20) nicht überwinden kann.

Um das Element (10) in einem nicht dargestellten Gewebe ablegen zu können, wirkt auf den Mandrin (26) ein irgendwie ausgebildetes Stiftelement mit einem Durchmesser kleiner als der Durchmesser des Lumens der Kanüle (20) derart, daß beim Zurückziehen dieser der Mandrin (24) in seiner Stellung verharrt, wodurch das Element (10) in das Gewebe gelangt. Hierbei kann das vordere Ende des Mandrins (24) mit dem Gewebe in Berührung kommen. Dies ist dann nicht schädlich, wenn der Mandrin (24) mit der Kanüle (12) und deren Halterung (16) eine wegweifbare Einheit bildet. Andernfalls hat eine Sterilisation zu erfolgen.

Das auf den Mandrin (24) einwirkende Stiftelement einer nicht dargestellten Handhabe kann ohne erforderliche Sterilisation immer wieder benutzt werden, da eine Berührung mit dem Gewebe nicht erfolgt.

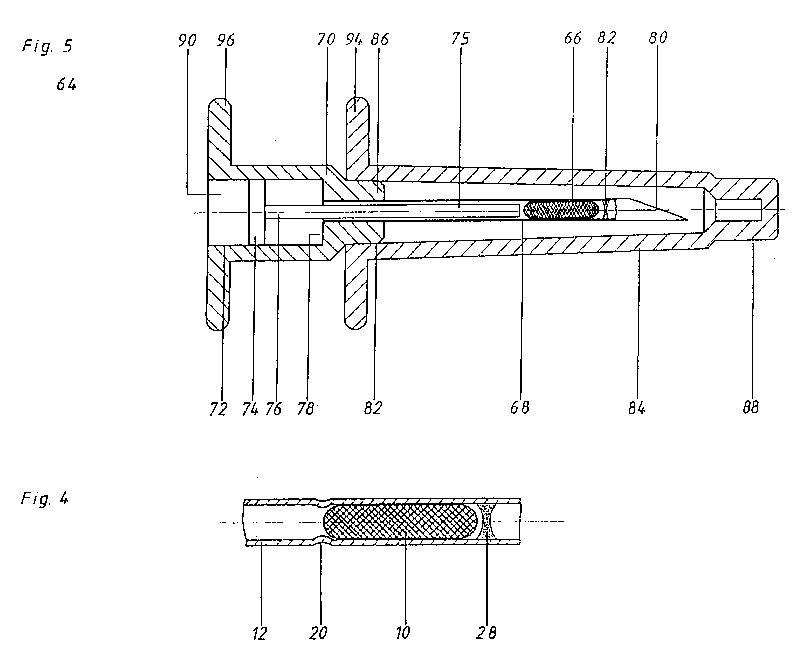

Wie anhand der Fig. 4 verdeutlicht werden soll, ist das Element (10) in der Kanüle (12) halterungsseitig von der Einschnürung (20) und kanülenspitzenseitig von einem Verschluß (28) lagefixiert, der durch einen Klebstofftropfen, der in die Kanüle hineingetropft wird, gebildet werden kann. Das Klebstoffmaterial weist dabei eine Adhäsion zu dem Kanülenmaterial auf, daß dann. wenn ein Durchdringen von dein Identifikationsträger (10) und dem nicht dargestellten Mandrin (24) erfolgt, ein Haften des Material nicht an diesem, sondern allein an der Kanülenwandung erfolgt. Das Klebstoffmaterial zieht sich dann zu einer Art Tropfenform zusammen.

Ist im Ausführungsbeispiel der Fig. 4 der Verschluß (28) querschnittsabdeckend dargestellt, so ist dies kein zwingendes Merkmal. Vielmehr reicht es, wenn der Verschluß durch eine ringförmig entlang der Innenwandung der Kanüle (12) verlaufende Klebstoffmaterialverstärkung erfolgt. Gegebenenfalls kann der Querschnitt auch durch eine dünne Haut aus Klebstoffmaterial verschlossen werden.

Alternativ wird jedoch der Verschluß (28) durch ein einen medizinischen Wirkstoff enthaltendes salbenartiges Material gebildet, das gleichfalls in die Kanüle hineingetropft werden kann. Das Material weist dabei eine Adhäsion zu dem Element (10) derart auf, daß ein Haften an diesem erfolgt. Hierdurch gelangt der medizinische Wirkstoff mit in das Gewebe, wodurch etwaigen Entzündungen vorgebeugt werden kann. Als Wirkstoff dient vorzugsweise ein Poly(1-vinyl-2-pyrolidon)-Jod-Komplex. Auch kann das Material in Form einer Salbe Polyäthylenglykol enthalten.

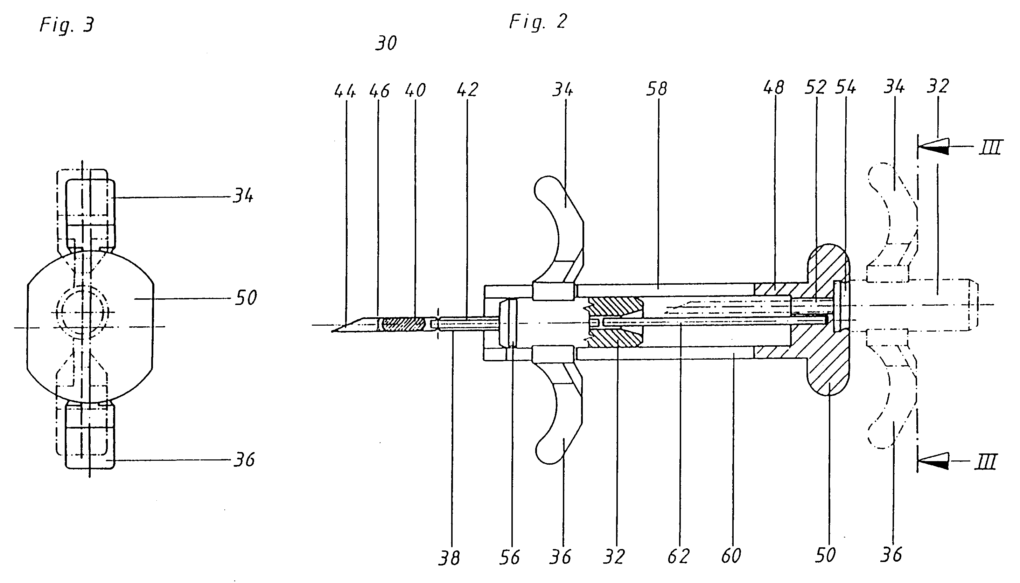

In den Fig. 2 und 3 ist ein Einmalimplantator (30) dargestellt. Der Einmalimplantator (30) weist eine Kanülenhalterung (32) mit abragenden Handhabungselementen (34) und (36) auf. Von der Kanülenhalterung (32) und diese zentral durchsetzend geht eine Kanüle (38) aus, die dem Aufbau der Kanüle (12) entspricht. Folglich ist in der Kanüle (38) ein Element (40) lagefixiert angeordnet. Die Lagefixierung erfolgt zum einen durch eine teilweise Querschnittsverringerung des Kanülenlumens und zum anderen durch einen im Abstand zur Kanülenspitze (44) angeordneten Tropfen bzw. Ring (46).

Die Kanüle (38) ist im unbenutzten Zustand von einer Schutzkappe (48) umgeben. Bei der Schutzkappe (48) handelt es sich um einen einseitig geschlossenen zylindrischen Körper, der kanülenspitzenseitig geöffnet ist. Der Boden- oder Basisabschnitt (50) der Schutzkappe (48) weist flanschartige Erstreckungen auf. Außermittig verläuft in dem Bodenabschnitt (50) eine Bohrung (52), die außenseitig in eine zylindrische Erweiterung (54) übergeht, deren Durchmesser dem Außendurchmesser der Kanülenhalterung (32) im sich an der Kanüle (40) anschließenden Bereich (56) entspricht. Dieser Bereich (56) kann dabei stufenförmige Abschnitte aufweisen, die sich in der Vertiefung (54) gleichfalls wiederfinden. Hierdurch kann eine Verrastung zwischen der Schutzkappe (48) und dem Kanülenhalter (32) dann erfolgen, wenn die Kanüle (38) innerhalb der Schutzkappe (48) verläuft, wenn also die Schutzkappe (48) die eigentliche Funktion eines Schutzes für die Kanüle (38) ausüben soll.

Der Innendurchmesser der Schutzkappe (48) enspricht dem Außendurchmesser des Kanülenhalters (32). Soll das Element (40) in einem Gewebe abgelegt werden, so wird die Handhabe (32) von vorne in die Schutzkappe (48) eingeführt. Hierzu sind in der Wandung der Schutzkappe (48) schlitzförmige Führungen (58) und (60) vorgesehen, deren jeweilige Breite der der Handhabungselemente (34) und (36) entspricht. Die Schlitze oder Führungen (58) und (60) zeigen dabei einen stufenförmigen Verlauf, um ein unbeabsichtigtes Herausgleiten des Kanülenhalters (32) mit der Kanüle (38) aus der Schutzkappe (48) zu vermeiden.

Von der Innenseite des Bodenabschnittes (52) geht ein Stiftelement wie Stahlstift (62) aus, der sich bei in der Schutzkappe (48) eingebrachter Kanülenhalterung (32) entlang der Längsachse der Kanüle (38) erstreckt, um in diese einzudringen. Der Durchmesser des Stiftes (62) ist dabei kleiner als der Innendurchmesser der Kanüle (38). Wird die Kanülenhalterung (32) über die Handhabungselemente (34) und (36) in Richtung des Schutzkappenbodens (48) gezogen, so dringt hierdurch der Stahlstift (62) in die Kanüle (38), um einen Mandrin (42) scheinbar in Richtung der Kanülenspitze (44) zu verschieben, wodurch das Element (40) mitgenommen und Somit in einem Gewebe abgelegt werden kann.

Die gestrichelte Darstellung des Kanülenhalters (32) mit den Handhabungselementen (34) und (36) soll die Position andeuten, in der die Schutzkappe (48) ihre eigentliche Funktion ausübt, also bei der die Kanüle die Durchbrechung (52) durchsetzt, um von der Schutzkappe (48) geschützt zu werden. Die Erstreckung der Kanüle innerhalb der Schutzkappe (48) ist dann geringer als die lichte Tiefe der Schutzkappe (48) selbst.

In Fig. 5 ist eine Schnittdarstellung durch eine weitere Ausführungsform eines Implantators (64) zum Implantieren eines Elements (66) wie Transponder dargestellt.

Der Implantator (64) umfaßt eine Kanüle (68), die von einem Kanülenhalter (70) aufgenommen ist, der seinerseits außerhalb der Kanüle (68) eine hohlzylindrische Erweiterung (72) aufweist, die nachstehend beschriebenerweise als Zylinder für einen Kolben wirkt.

Innerhalb der Kanüle (68) befindet sich ein verschiebbarer Mandrin (75), der außenseitig einen dem Innendurchmesser der hohlzylindrischen Erweiterung (72) angepaßten tellerförmig ausgebildeten Abschnitt (74) aufweist, der folglich der Kolben ist. Der von dem Kolben (74) ausgehende innerhalb der Kanüle (68) verschiebbare Abschnitt (76), also quasi die Kolbenstange, weist eine Länge auf, die ausreicht, um bei bis zum Boden (78) der hohlzylindrischen Erweiterung (72) geschobenen Kolben (74) das Element (66) wie Transponder, medizinisches Präparat oder ähnliches über die Öffnung (80) der Kanüle (68) in einem Gewebe ablegen zu können.

Das Element (66) ist dabei mittels eines im Bereich der Öffnung (80) der schräg angeschlieffenen Kanüle (68) vorhandenen Silikonpfropfens, Klebstofftropfens (82), medizinischen Wirkstoff enthaltendes viskoses Material oder ähnliches fixiert.

Außenseitig ist die Kanüle (68) von einer Schutzkappe (84) abgedeckt, die auf einen die Kanüle (68) umgebenden verjüngten Abschnitt (86) des Kanülenhalters (70) schiebbar ist. Schutzkappe (84) und Kanülenhalter (70) können dabei einstückig und im Bereich der Verjüngung (86) mit einer Sollbruchstelle ausgebildet sein, um ein ungewolltes Entfernen der Schutzkappe (84) zu verhindern, wodurch gleichzeitig überprüft werden kann, ob die Kanüle noch steril ist oder nicht.

Erfindungsgemäß nimmt die Schutzkappe (84) nicht nur die Funktion des Schutzes der Kanüle (68) wahr, sondern kann gleichzeitig als Hilfswerkzeug zum Verschieben des Mandrins (75) in Richtung der Kanülenöffnung (80) dienen, um also das Element (66) in das Gewebe zu schieben.

Die Schutzkappe (84) weist eine Zylinderform auf, deren Außendurchmesser dem Innendurchmesser der hohlzylindrischen Erweiterung (72) angepaßt ist. Die Schutzkappe (84) kann folglich die Funktion eines Stempels ausüben, um auf den Kolben (74) und damit auf den Mandrin (75) zu dessen Verschieben innerhalb der Kanüle (68) zu wirken.

Um ein einfaches Eindringen der Schutzkappe (84) in die hohlzylindrische Erweiterung (72) zu ermöglichen, weist die Schutzkappe (84) in ihrem geschlossen Bereich einen verjüngten Abschnitt (88) auf.

Um das Verschieben der Schutzkappe (84) in die hohlzylindrische Erweiterung (72) zu erleichtern, um also Kanülenträger (70) und Schutzkappe (84) bei deren Verschieben zueinander leichter erfassen zu können, weisen die Öffnung (90) des Kanülenhalters (70) bzw. die Öffnung (92) der Schutzkappe (84) nach außen abgewinkelte Randabschnitte (94) und (96) auf, die jedoch nicht umlaufend ausgebildet sein müssen. Vielmehr reichen diametral gegenüberliegend nach außen gerichtete Abschnitte aus.

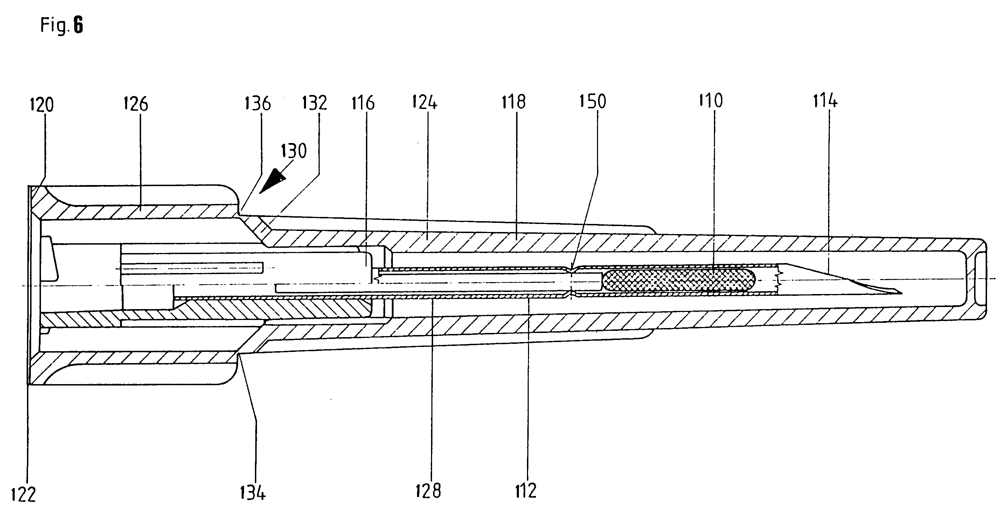

Anhand der Fig. 6 bis 9 sollen besonders hervorzuhebende Ausbildungen von Schutzkappen erläutert werden, die gleichzeitig die Funktion einer sterilen Verpackung ausüben.

Bei der in Fig. 6 dargestellten Kanüle (112) kann es sich ebenfalls um eine Stahlkanüle, Kunststoffkanüle oder um eine Kanüle anderen geeigneten Materials mit schräg angeschliffener Spitze (114) handeln. Die Kanüle (112) wird von einem Kanülenhalter (116) aufgenommen. Lösbar mit dem Kanülenhalter ist eine Schutzkappe (118) vorgesehen, die die Kanüle (112) über ihre gesamte Länge umgibt, diese also aufnimmt.

In der Kanüle (112) ist ein in einem Gewebe abzulegendes Element (110) lagefixiert bzw. verschiebbar angeordnet.

Bei der Schutzkappe (118) handelt es sich folglich um einen hohlzylinderförmigen Körper, innerhalb dessen sich die Kanüle (112) einschließlich deren Kanülenhalter (116) erstreckt. Die Schutzkappe (118) ist in ihrem freien Stirnbereich (120) von einer flexiblen Abdeckung wie Papier (122) verschlossen. Folglich ergibt die Schutzkappe mit der Abdeckung (122) eine Verpackung für die Kanüle (112).

Die Schutzkappe (118) setzt sich aus zwei Abschnitten (124) und (126) zusammen. Der vordere Abschnitt (124) umgibt den freien Bereich (128) der Kanüle (112) und ist lösbar auf dem Kanülenhalter (118) angeordnet. Folglich weist der Abschnitt (124) in dem den Kanülenhalter (116) umgebenden Bereich einen Innenquerschnitt auf, der dem Außenquerschnitt des Kanülenhalters (116) angepaßt ist.

Der Abschnitt (124) kann sich zur Kanülenspitze (114) hin verjüngen. Auch kann in dem Übergangsbereich von dem den Kanülenhalter (116) umgebenden Bereich zu dem die freie Kanüle (128) umgebenden Bereich eine Stufe und eine damit verbundene Querschnittsveränderung ausgebildet sein.

Der zweite, der Kanülenspitze (114) abgewandte Abschnitt (126) der Schutzkappe (118) weist im Vergleich zu dem ersten Abschnitt (124) einen größeren Querschnitt und damit eine größere lichte Weite auf. Damit umgibt der Abschnitt (126) den Kanülenhalter (116) beabstandet.

Um insbesondere den Abschnitt (126) bei noch auf dem Kanülenhalter (116) verbleibendem Abschnitt (124) entfernen zu können, ist im Übergangsbereich eine Sollbruchstelle ausgebildet, die in der Fig. 6 mit dem Bezugszeichen (130) versehen ist. In diesem Bereich weist der vordere Abschnitt (124) eine konische Erweiterung (132) auf. Der Durchmesser des freien äußeren Randes (134) der konischen Erweiterung (132) ist nun in etwa gleich dem Innendurchmesser des sich anschließenden zweiten Abschnitts (126) der Schutzkappe (118). Hierdurch bildet sich eine umlaufende Abreißkante (136) aus, die die Funktion der Sollbruchstelle zeigt.

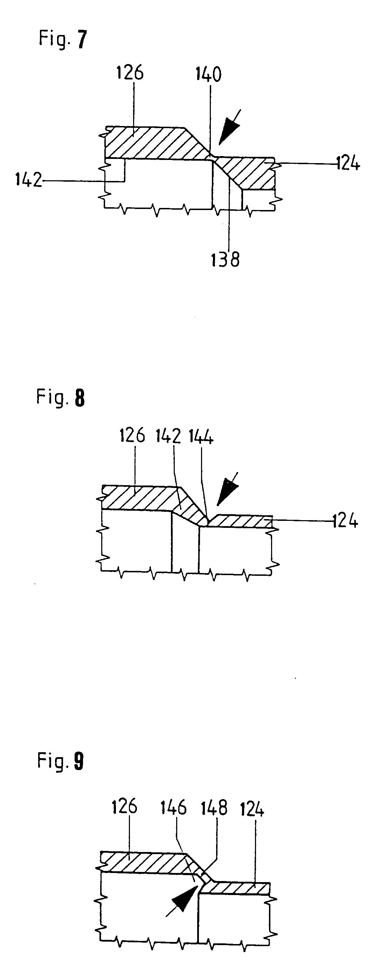

Selbstverständlich ist es nicht erforderlich, daß der vordere Abschnitt (124) der Schutzkappe (118) die konische Erweiterung (132) zeigt. Vielmehr kann der Abschnitt (124) in seinem dem Endabschnitt (126) zugewandten Stirnbereich (38) (Fig. 7) angeschrägt sein, um auf diese Weise einen stegartigen Übergangsbereich (114) zu der Innenwandfläche (142) des Abschnittes (126) auszubilden. Auch auf diese Weise bildet sich eine Sollbruchstelle in Form einer umlaufenden stegartigen Wandung aus, deren Stärke vom der Differenz zwischen dem Außendurchmesser des Abschnitts (124) und dem Innendurchmesser des Abschnitts (126) der Schutzkappe (118) abhängt.

Den Fig. 8 und 9 sind weitere Ausbildungen von Sollbruchstellen zwischen dem vorderen Abschnitt (124) und dem hinteren Abschnitt (126) zu entnehmen.

So ist nach dem Ausführungsbeispiel der Fig. 8 im Abstand zum Übergangsbereich (144) zwischen den Abschnitten (124) und (126) eine umlaufende Einschnürung oder Einkerbung (144) in der Wandung des Abschnitts (124) vorgesehen.

Nach der Fig. 8 erstreckt sich vom dem Abschnitt (124) zugewandten Stirnbereich (146) des hinteren Abschnitts (126) eine stegartig ausgebildete Verbindungswandung (148) zum Abschnitt (124) hin, die gleichfalls die Funktion einer umlaufenden Abreißkante und damit Sollbruchstelle ausübt.

Durch die erfindungsgemäße Lehre übt die Schutzkappe (118) eine Doppelfunktion aus. So dient sie einerseits in üblicher Weise als Schutz für die Kanülenspitze (114). Gleichzeitig ist die Schutzkappe (118) Einwegverpackung, da es nur noch erforderlich ist, die frei hintere Stirnfläche mit einem vorzugsweise flexiblen Material wie Papier zu verschließen, nachdem die Kanüle in die Schutzkappe (118) eingebracht worden ist. Trotz dieser Doppelfunktion wird jedoch die Handhabung der Kanüle nicht beeinträchtigt, da durch die Ausbildung der Sollbruchstellen ein leichtes Entfernen des hinteren Teils der Schutzkappe, also des Abschnitts (126) möglich ist, um somit die Kanüle mit dem Kanülenhalter z. B. in einer pistolenartigen Handhabung festzulegen.

Wie der Fig. 6 des weiteren zu entnehmen ist, weist - wie bei den zuvor beschriebenen Ausführungsbeispielen - die Kanüle (112) im Abstand einerseits zur Spitze (114) und andererseits zur Kanülenhalterung (116) eine Lumeneinschnürung (150) auf, die z. B. durch in bezug auf die Kanülenlängsachse zwei diametral angeordnete Körnungen oder eine zumindest bereichsweise umlaufende Sicke gebildet werden kann. Der Querschnitt im Bereich der Einschnürung (150) ist dabei geringer als der Durchmesser des Elementes (110), wodurch sichergestellt ist, daß dieses über die Einschnürung (150) hinaus nicht in Richtung der Kanülenhalterung (116) verrutschen kann.

In der Kanüle (112) ist des weiteren ein nicht dargestellter Mandrin verschiebbar angeordnet, und zwar von der Kanülenhalterungsseite her. Der Mandrin hat im der Kanülenspitze (114) abgewandten Endbereich einen verstärkten Querschnitt wie umlaufenden Wulst, dessen Durchmesser größer als der der Einschnürung (150) ist. Hierdurch ist sichergestellt, daß die Verstärkung die Einschnürung (150) nicht überwinden kann.

Um das Element (110) in einem nichtdargestellten Gewebe ablegen zu können, wirkt auf den Mandrin ein irgendwie ausgebildetes Stiftelement mit einem Durchmesser kleiner als der des Lumens der Kanüle (112) derart, daß beim Zurückziehen dieser der Mandrin in seiner Stellung verharrt, wodurch das Element (110) in dem Gewebe abgelegt werden kann.

Zur weiteren Lagefixierung des Elementes (110) zwischen der Einschnürung (150) und der Kanülenspitze (114) kann zwischen dem Mandrin und der Kanülenspitze (114) ein nicht dargestellter Verschlußpfropfen vorgesehen sein, der beim Ablegen des Elementes (110) entfernt und/oder teilweise mitgenommen wird. Bei dem Verschlußpfropfen kann es sich z. B. um einen Silikonpfropfen oder auch um ein medizinisches Präparat handeln, um gegebenenfalls bei beim Implantieren auftretende Verletzungen des Gewebes eine Heilwirkung hervorzurufen.

## Patentansprüche

1. Kanüle (12, 38, 68, 112) mit am vorderen Ende angeschliffener Spitze (14, 44, 80, 114) und am hinteren Ende die Kanüle haltendem Kanülenhalter, einem in der Kanüle verschiebbaren Mandrin (24, 42, 75) sowie gegebenenfalls einer lösbar mit dem Kanülenhalter (16, 32, 70, 116) verbundenen, die Kanülenspitze umgebenden Schutzkappe, (18, 48, 84, 118) vorzugsweise in Form eines einseitig offenen Hohlzylinders, wobei die Kanüle bestimmt ist zum Ablegen eines Elementes (10, 40, 66, 110) in einem Körper eines Lebewesens durch Wechselwirken mit und Relativbewegung zwischen dem Mandrin und der Kanüle, wobei das Element vor Ablegen in dem Körper zwischen dem Mandrin (24, 42, 75) und der Kanülenspitze (14, 44, 80, 114) lagefixiert ist,
**dadurch gekennzeichnet**,
daß das Element (10, 40, 66, 110) kanülenspitzenseitig von einem die Kanüle (12, 38, 68, 112) zumindest bereichsweise verschließenden Verschluß (28, 46, 82) in Form eines einen medizinischen Wirkstoff enthaltenden salbenartigen Materials, eines Klebstoffmaterials oder eines Silikonpfropfens und/oder halterungsseitig durch eine Querschnittsänderung (20, 150) der Kanüle (12, 38, 68, 112) lagefixiert ist.

2. Kanüle nach Anspruch 1,
**dadurch gekennzeichnet**,
daß der Verschluß (28, 46, 82) im Abstand zur Kanülenspitze (14, 44, 80, 114) angeordnet ist.

3. Kanüle nach Anspruch 1,
**dadurch gekennzeichnet**,
daß das Klebstoffmaterial in bezug auf die Kanüle (12, 38, 68, 112) eine Adhäsion aufweist, die beim Durchdringen des Klebstoffmaterials von dem Element (10, 40, 66, 110) ein Haften an der Kanülenwandung sicherstellt.

4. Kanüle nach Anspruch 1,
**dadurch gekennzeichnet**,
daß das salbenartige Material wie medizinische Salbe einen Poly-Jod-Komplex als Wirkstoff enthält.

5. Kanüle nach Anspruch 1,
**dadurch gekennzeichnet**,
daß das salbenartige Material beim Ablegen des Elementes (10, 40, 66, 110) zumindest bereichsweise an diesem haftet.

6. Kanüle nach Anspruch 1,
**dadurch gekennzeichnet**,
daß die halterungsseitige Querschnittsveränderung (20, 150) zur Lagefixierung des Elementes (10, 40, 66, 110) durch vorzugsweise zumindest zwei diametral zur Längsachse der Kanüle (12, 38, 68, 112) verlaufende Einbuchtungen oder eine zumindest abschnittsweise umlaufende Sicke erfolgt, wobei die Querschnittsveränderung wie Querschnittsverringerung vorzugsweise als Schiebewegbegrenzung für der Kanülenspitze (14, 44, 80, 114) abgewandtem Endbereich des Mandrins (24, 42, 75) ausgebildet ist.

7. Kanüle nach Anspruch 1,
**dadurch gekennzeichnet**,
daß sich die Kanüle zusammen mit der Kanülenhalterung (116) vollständig innerhalb der Schutzkappe (118) erstreckt, die an ihrem offenen Ende (120) mit einer flexiblen Abdeckung (122) versehen ist, daß die Schutzkappe zumindest zwei Abschnitte (124, 126) unterschiedlichen Querschnitts aufweist, von denen ein vorderer (erster) die Kanülenspitze (114) umgebender Abschnitt (124) mit seinem der Kanülenspitze abgewandten Bereich auf dem Kanülenhalter (116) angeordnet ist, und daß ein endseitiger (zweiter) Abschnitt (126) einerseits mit der flexiblen Abdeckung (122) verschlossen ist und andererseits abtrennbar mit dem ersten Abschnitt verbunden ist.

8. Kanüle nach Anspruch 7,
**dadurch gekennzeichnet**,
daß zwischen dem ersten Abschnitt (124) und dem zweiten den Kanülenhalter beabstandet umgebenden Abschnitt (126) der Schutzkappe (118) eine Sollbruchstelle (136, 140, 144, 148) ausgebildet ist.

9. Kanüle nach Anspruch 8,
**dadurch gekennzeichnet**,
daß zur Ausbildung der Sollbruchstelle (148) vom dem ersten Abschnitt (124) zugewandten Stirnbereich des zweiten Abschnitts (126) eine stegartige den ersten mit dem zweiten Abschnitt verbindende Wandung (146) ausgeht.

10. Kanüle nach Anspruche 8,
**dadurch gekennzeichnet**,
daß im Verbindungsbereich (136, 140) zwischen dem ersten und dem zweiten Abschnitt (124, 126) der Schutzkappe (118) der Außendurchmesser des ersten Abschnitts in etwa dem Innendurchmesser des zweiten Abschnitts entspricht.

11. Kanüle nach Anspruch 8,
**dadurch gekennzeichnet**,
daß im Übergangsbereich zwischen dem ersten und dem zweiten Abschnitt (124, 126) der Schutzkappe (118) vorzugsweise in dem ersten Abschnitt eine umlaufende Einschnürung wie Einkerbung (144) zur Ausbildung der Sollbruchstelle verläuft.

12. Kanüle insbesondere nach Anspruch 1,
**dadurch gekennzeichnet**,
daß das Element (10, 40, 66, 110) ein Identifikationsträger, ein medizinisches Langzeitpräparat, eine radioaktive Substanz enthaltende Kapsel oder ein metallenes Kontrastelement ist oder enthält.

13. Kanüle nach Anspruch 1,
**dadurch gekennzeichnet**,
daß zum Verschieben des Mandrins (42) in Richtung der Kanülenspitze (44) ein Stiftelement wie Stahlstift (62) in die Kanüle (38) einführbar ist, das vorzugsweise von einer Bodenfläche der Schutzkappe (48) ausgeht.

14. Kanüle nach Anspruch 1,
**dadurch gekennzeichnet**,
daß die Schutzkappe (48) ein einseitig offener Hohlzylinder ist, daß der Innendurchmesser der Schutzkappe dem Außendurchmesser des Kanülenhalters (32) entspricht und daß entlang der Längsachse der Schutzkappe von dessen innenseitiger Bodenfläche ausgehend das Stiftelement (62) abragt, das bei Verschieben der Kanüle (38) in Richtung der Bodenfläche mit dem Mandrin (24) derart wechselwirkt, daß dieser relativ zur Kanüle (12, 38) verschiebbar ist.

15. Kanüle nach Anspruch 14,
**dadurch gekennzeichnet**,
daß von dem Kanülenhalter (32) seitliche Handhabungselemente (34, 36) abragen, die in Längsschlitzen (58, 60) der Schutzkappe (48) führbar sind, wobei der jeweilige Längsschlitz (58, 60) vorzugsweise zwei versetzt zueinander verlaufende Abschnitte aufweist.

16. Kanüle nach Anspruch 14,
**dadurch gekennzeichnet**,
daß die Bodenwandung (50) der Schutzkappe (48) eine außermittig verlaufende Durchbrechung (52) zum Durchsetzen der Kanüle (38) aufweist.

17. Kanüle nach einem der vorangegangenen Ansprüche,
**dadurch gekennzeichnet**,
daß der Kanülenhalter (70) im sich an die Kanüle (68) anschließenden Bereich eine hohlzylindrische Erweiterung (72) aufweist, an deren Innendurchmesser ein tellerförmig ausgebildeter Abschnitt (74) des Mandrins (75) angepaßt ist, und daß die hohlzylindrische Erweiterung zur verschiebbaren Aufnahme eines Schiebeelementes (84) ausgebildet ist.

18. Kanüle nach Anspruch 16,
**dadurch gekennzeichnet**,
daß das Schiebeelement die Schutzkappe (84) ist.

19. Kanüle nach Anspruch 17,
**dadurch gekennzeichnet**,
daß die Schutzkappe (84) im wesentlichen eine Zylinderform aufweist, deren Außendurchmesser zumindest bereichsweise dem Innendurchmesser der hohlzylindrischen Erweiterung (72) des Kanülenhalters (70) angepaßt ist.

20. Kanüle nach Anspruch 18,
**dadurch gekennzeichnet**,
daß der Kanülenhalter (70) in seinem geschlossenen Endbereich (86) einen geringeren Durchmesser als im dem Innendurchmesser der hohlzylindrischen Erweiterung (72) angepaßten Bereich aufweist.

21. Kanüle nach zumindest Anspruch 17,
**dadurch gekennzeichnet**,
daß das geschlossene Ende (88) der Schutzkappe (84) zumindest bereichsweise komplementär zur der außenliegenden Fläche des tellerförmigen Abschnitts (74) des Mandrins (75) ausgebildet ist.

## Claims

1. Needle (12, 38, 68, 112) with a sharpened point (14, 44, 80, 114) at the tip and a needle holder at the rear end holding the needle, a mandrel (24, 42, 75) which can be displaced inside the needle, and optionally a protective cap (18, 48, 84, 118) enclosing the needle point and detachably attached to the needle holder (16, 32, 70, 116), preferably in the form of a hollow cylinder open at one end, whereas the needle is intended to insert an object (10, 40, 66, 110) into the body of a living being by interaction and relative movement of the mandrel and needle, whereas the object is fixed in position, before insertion in the body, between the mandrel (24, 42, 75) and the needle point (14, 44, 80, 114),
**characterized in**
that the object (10, 40, 66, 110) is fixed in position at the needle end by a closure (28, 46, 82) which closes off at least a region of the needle (12, 38, 68, 112) in the form of an ointment-like material containing a medical active ingredient, an adhesive material or a silicon stopper, and/or the object is fixed in position at the holder end by means of a change in the cross section (20, 150) of the needle (12, 38, 68, 112).

2. Needle according to Claim 1,
**characterized in**
that the closure (28, 46, 82) is arranged at a distance from the needle point (14, 44, 80, 114).

3. Needle according to Claim 1,
**characterized in**
that the adhesive material has, with respect to the needle (12, 38, 68, 112) adhesive properties ensuring adhesion to the needle wall when the adhesive material is penetrated by the object (10, 40, 66, 110).

4. Needle according to Claim 1,
**characterized in**
that the ointment-like material, such as medicinal ointment, preferably contains a polyiodine complex as active ingredient.

5. Needle according to Claim 1,
**characterized in**
that the ointment-like material adheres at least in certain areas to the object (10, 40, 66, 110) when it is inserted.

6. Needle according to Claim 1,
**characterized in**
that the change in cross section (20, 150) at the holder end for fixing the object (10, 40, 66, 110) in position is implemented by preferably at least two indentations extending diametrically to the long axis of the needle (12, 38, 68, 112) or an at least partially circumferential bead, whereas the change in cross section such as a reduction in cross section is preferably designed as a limitation of displacement travel for the end region of the mandrel (24, 42, 75) facing away from the needle point (14, 44, 80, 114).

7. Needle according to Claim 1,
**characterized in**
that the needle, together with the needle holder (116), extends completely inside the protective cap (118), which is provided at its open end (120) with a flexible covering (122), that the protective cap has at least two sections (124, 126) of different cross sections, of which a front (first) section (124) enclosing the needle point (114) is arranged with its region facing away from the needle point on the needle holder (116), and that an end (second) section (126) is on the one hand sealed with a flexible covering (122) and on the other hand separably attached to the first section.

8. Needle according to Claim 7,
**characterized in**
that a defined break point (136, 140, 144, 148) is configured between the first section (124) and the second section (126), surrounding the needle holder at a distance, of the protective cap (118).

9. Needle according to Claim 8,
**characterized in**
that to form the defined break point (148), a partiation (146) attaching the first section (124) to the second section (126) extends from the end region of the second section that faces the first section.

10. Needle according to Claim 8,
**characterized in**
that in the attachment region (136, 140) between the first and second sections (124, 126) of the protective cap (118), the outside diameter of the first section corresponds approximately to the inside diameter of the second section.

11. Needle according to Claim 8,
**characterized in**
that a circumferential constriction such as a notch (144) extends, preferably in the first section, in the transition region between the first and second sections (124, 126) of the protective cap (118), to form the defined break point.

12. Needle in particular according to Claim 1,
**characterized in**
that the object (10, 40, 66, 110) is or contains an identification carrier, a long-term medicinal preparation, a capsule containing a radioactive substance, or a metallic contrast element.

13. Needle according to Claim 1,
**characterized in**
that in order to displace the mandrel (42) toward the needle point (44), a pin element such as a steel pin (62), which preferably extends from a base surface of the protective cap (48), can be introduced into the needle (38).

14. Needle according to Claim 1,
**characterized in**
that the protective cap (48) is a hollow cylinder open at one end; that the inside diameter of the protective cap corresponds to the outside diameter of the needle holder (32); and that the pin element (62) projects along the long axis of the protective cap, extending from its inside base surface, and, when the needle (38) is displaced toward the base surface, interacts with the mandrel (24) so that the latter can be displaced relative to the needle (12, 38).

15. Needle according to Claim 14,
**characterized in**
that lateral handle elements (34, 36), guidable in lengthwise slits (58, 60) of the protective cap (48), project from the needle holder (32), each lengthwise slit (58, 60) preferably having two sections extending at an offset to one another.

16. Needle according to Claim 14,
**characterized in**
that the bottom wall (50) of the protective cap (48) has a perforation (52) extending eccentrically, to allow passage of the needle (38).

17. Needle according to one of the preceding claims,
**characterized in**
that the needle holder (70) has, in the region adjacent to the needle (68), a hollow cylindrical enlargement (72) to whose inside diameter a plate-shaped section (74) of the mandrel (75) is matched; and that the hollow cylindrical enlargement is designed to displaceably receive a sliding element (84).

18. Needle according to Claim 16,
**characterized in**
that the sliding element is the protective cap (84).

19. Needle according to Claim 17,
**characterized in**
that the protective cap (84) has substantially the shape of a cylinder, the inside diameter of which is matched, at least in certain regions, to the outside diameter of the hollow cylindrical enlargement (72) of the needle holder (70).

20. Needle according to Claim 18,
**characterized in**
that the needle holder (70) has in its closed end region (86) a smaller diameter than in the region matched to the inside diameter of the hollow cylindrical enlargement (72).

21. Needle according to at least Claim 17,
**characterized in**
that the closed end (88) of the protective cap (84) is designed, at least in certain regions, to be complementary to the external surface of the plate-shaped section (74) of the mandrel (75).

## Revendications

1. Canule (12, 38, 68, 112) avec à son extrémité avant une pointe affutée (14, 44, 80, 114) et à l'extrémité arrière une pièce d'appui (porte-canule) maintenant la canule, un mandrin (24, 42, 75) pouvant coulisser dans la canule ainsi qu'éventuellement un capot protecteur (18, 48, 74, 118) lié au porte-canule (16, 32, 70, 116) mais détachable, entourant la canule, de préférence sous la forme d'un cylindre creux ouvert d'un côté, tandis que la canule est destinée à injecter un élément (10, 40, 66, 110) dans un corps d'un être vivant par interaction et mouvement relatif entre le mandrin et la canule, l'élément avant injection dans le corps étant fixé en position entre le mandrin (24, 42, 75) et la pointe de canule (14, 44, 80, 114), caractérisée en ce que l'élément (10, 40, 66, 110) est maintenu en position du côté de la pointe de la canule par un dispositif obturateur (28, 46, 82) bloquant au moins localement la canule (12, 38, 68, 112) sous la forme d'un matériau pâteux contenant une substance active médicinale, d'un matériau adhésif ou d'un tampon de silicone et/ou du côté appui par un changement de section transversale (20, 150) de la canule (12, 38, 68, 112).

2. Canule selon la revendication 1, caractérisée en ce que l'obturateur (28, 46, 82) est placé à une certaine distance par rapport à la pointe de la canule (14, 44, 80, 114).

3. Canule selon la revendication 1, caractérisée en ce que le matériau adhésif comporte un pouvoir d'adhésion par rapport à la canule (12, 38, 68, 112) qui lors du passage de l'élément (10, 40, 66, 110) à travers le matériau adhésif assure une adhérence sur la paroi de la canule.

4. Canule selon la revendication 1, caractérisée en ce que le matériau pâteux comme pâte médicinale contient un complexe poly-iode en tant que substance active.

5. Canule selon la revendication 1, caractérisée en ce que le matériau pâteux lors de l'insertion de l'élément (10, 40, 66, 110) adhère au moins localement à celui-ci.

6. Canule selon la revendication 1, caractérisée en ce que le changement de section transversale (20, 150) du côté appui pour le blocage en position de l'élément (10, 40, 66, 110) est produit de préférence par au moins deux dentelures se développant diamétralement par rapport à l'axe longitudinal de la canule (12, 38, 68, 112) ou par une moulure circulaire au moins sur un segment, le changement de section transversale étant réalisé comme resserrement de cette surface, de préférence en tant que limitation au mouvement de coulissement de la zone terminale du mandrin (24, 42, 75) opposée à la pointe de canule (14, 44, 80, 114).

7. Canule selon la revendication 1, caractérisée en ce que :
- la canule, conjointement avec le porte-canule (116) s'étend complètement à l'intérieur du capot protecteur (118), qui est muni à son extrémité ouverte (120) d'un recouvrement flexible (122).
- Le capot protecteur comporte au moins deux segments (124, 126) différents, parmi lesquels un segment avant (124) (le premier) entourant la pointe de la canule (114) est placé avec sa zone opposée à la pointe de la canule sur le porte-canule (116) et
- un segment (126) du côté de l'extrémité (le deuxième) est fermé d'une part par le recouvrement flexible (122) et d'autre part est relié au premier segment, mais en est séparable.

8. Canule selon la revendication 7, caractérisée en ce que entre le premier segment (124) et le deuxième segment (126) du capot de protection entourant à une certaine distance le porte-canule (118) est constitué d'un endroit de rupture de consigne (136, 140, 144, 148).

9. Canule selon la revendication 8, caractérisée en ce que pour la constitution de l'endroit de rupture (148) part de la zone frontale du deuxième segment (126) tournée vers le premier segment (124), une paroi (146) du type traverse reliant le premier au deuxième segment.

10. Canule selon la revendication 8, caractérisée en ce que dans la zone de liaison (136, 140) entre le premier et le deuxième segment (124, 126) du capot de protection (118) le diamètre extérieur du premier segment correspond à peu près au diamètre intérieur du deuxième segment.

11. Canule selon la revendication 8, caractérisée en ce que dans la zone de transition (124, 126) entre le premier et le deuxième segment du capot de protection (118), se développe de préférence, dans le premier segment, une gorge circulaire comme une entaille (144) pour la réalisation de l'endroit de rupture de consigne.

12. Canule en particulier selon la revendication 1, caractérisée en ce que l'élément (10, 40, 66, 110) est ou contient une préparation médicinale de longue durée, une capsule contenant une substance radioactive ou un élément de contraste métallique.

13. Canule selon la revendication 1, caractérisée en ce que pour le coulissement du mandrin (42) en direction de la pointe de la canule (44) un élément de tige comme une tige d'acier (62) peut être introduit dans la canule (38), élément qui sort d'une face du fond du capot de protection (48).

14. Canule selon la revendication 1, caractérisée en ce que :
- le capot de protection (48) est un cylindre creux ouvert d'un côté, le diamètre intérieur du capot de protection correspond au diamètre extérieur du porte-canule (32) et,
- l'élément de tige (62) se dresse le long de l'axe longitudinal du capot protecteur au sortir de la face du fond intérieur, élément qui lors du déplacement de la canule (38) en direction de la face du fond interagit avec le mandrin (24) de manière que celui-ci puisse coulisser par rapport à la canule (12, 38).

15. Canule selon la revendication 14, caractérisée en ce que des éléments de manipulation latéraux (34, 36) dépassent du porte-canule (32), éléments qui sont guidées dans des rainures longitudinales (58, 60) du capot de protection (48), la rainure longitudinale respective (58, 60) comportant de préférence deux segments se développant de manière décalée l'un par rapport à l'autre.

16. Canule selon la revendication 14, caractérisée en ce que la paroi de fond (50) du capot de protection (48) comporte une découpure (52) se développant de manière excentrique pour le chargement de la canule (38).

17. Canule selon une des revendications précédentes, caractérisée en ce que :
- le porte-canule (70) comporte dans la zone se raccordant à la canule (68) un élargissement (72) cylindrique et creux, sur le diamètre intérieur duquel est ajusté un segment (74) du mandrin (75) réalisé en forme de disque et,
- l'élargissement cylindrique creux est réalisé pour recevoir, avec déplacement possible, un élément coulissant (84).

18. Canule selon la revendication 16, caractérisée en ce que, l'élément coulissant est le capot de protection (84).

19. Canule selon la revendication 17, caractérisée en ce que, le capot de protection (84) a essentiellement une forme cylindrique, dont le diamètre extérieur est adapté en une zone au diamètre intérieur de l'élargissement (72) cylindrique creux du porte-canule (70).

20. Canule selon la revendication 18, caractérisée en ce que le porte-canule (70) comporte dans sa zone terminale fermée -(86) un diamètre plus faible que dans la zone adaptée au diamètre intérieur de l'élargissement (72) cylindrique creux.

21. Canule selon au moins la revendication 17, caractérisée en ce que l'extrémité fermée (88) du capot de protection (84) est réalisée au moins en une zone en complément à la surface du mandrin (75) située à l'extérieur du segment en forme de disque (74).
